# EUROPEAN PATENT APPLICATION

(11) **EP 1 208 805 A1**
(43) Date of publication of application: **29.05.2002**
(21) Application number: 99924049.2
(22) Date of filing: 24.05.1999
(51) Int. Cl.: A61B 17/72, A61B 17/17, A61B 17/16

(54) **NOVEL INTRAMEDULLARY NAIL FIXED TO THE BONE BY BOLTS WITHOUT REAMING AND MORE RESISTANT NAIL REQUIRING REAMING**

(71) Applicant: Colchero Rozas, Fernando, Mexico, D.F. 03100 (MX)
(72) Inventor: Colchero Rozas, Fernando, Mexico, D.F. 03100 (MX)
(74) Representative: EGLI-EUROPEAN PATENT ATTORNEYS
(86) International application number: MX9900014
(87) International publication number: WO0071041

(57) **Abstract**

The invention relates to a system for the treatment of bone fractures, lack of union in fractures, worst union and dismetries in tibia, femur and humerus. Said system is constituted by a nail to be used without milling of the pith channel, but also, when a greater diameter is involved, with milling thereof. It is preferably made up from stainless steel 316 LS or titanium alloys, with a broad proximal portion having two orifices and a body, and a distal narrower portion having a distal notch instead of an orifice. Bolts are passed through said proximal orifices and through the distal notch a greater diameter bolt. Conical screws of different lengths and diameters pass, once the nail is positioned within the pith channel, between the cortical of the bone and the nail, very tightened to both of them, thus increasing substantially the stability.

The instrumental to implant this novel nail comprises three elements: an instrument stabilizing the nail and the piece to find out the nail orifices and notch; a piece to find out the nail orifices and notch and an instrument showing exactly where the nail is located laterally. With said instrumental the orifices and the notch of the nail are easily located without a need for an image intensifier or radiographies.

It is the object of the invention to provide an osteosynthesis system with blocked intramedular nail, without pith milling or with a milling to locate the orifices and notch of the nail, and an highly firm additional fixing to fill completely the pith channel. When a highly resistant nail is needed for an obese person, this system also provides the above advantages, together with pith milling, whereby the surgery can be made with open focus.

## Description

### BACKGROUND OF THE INVENTION

Intramedular nails to be employed in fractures and the complications thereof go back a number of years from today. Formerly and up to the end of 60's, said nails were introduced within the pith channel in long bones of a patient without fastening of any kind to the bone. So, they were free within said pith channel, making thus that only those injuries at the bone neck, transversal or slightly oblique thereto, were capable of become stable to consolidate. This is due to the fact that said pith channel is broad at both the upper and lower ends with a neck at the middle portion thereof; whilst said nail presents the same diameter along the length thereof, whereby it remains separated apart from the bone cortical at the broadened portions and joined thereto at said neck.

Starting from 1968 Küntscher designs an embodiment wherein orifices are made transversal to the nail axis, so distributed that a couple of them are located above the fracture and another couple under said fracture. Screws are passed therethrough traversing also the bone, so that said bone, nail and screws become a sole structure. It is a drawback of these Küntscher nails that they are of weak strength due to the fact that they are hollow and with a thin wall made of stainless steel. The consequence of this fact is that the patient cannot walk by himself (herself) in order to prevent the nail to become broken, in case the injury is at the tibia or femur. The embodiment of the hollow nails has been practiced up to the present days, leaving aside the fragility thereof and the inconveniences for the patients, who must postpone their walking for weeks. Also this type of nails slow downs the time of consolidation due to the lack of function for the treated portion. Today it is well accepted that function is a basic fact for normal union of bone fractures and their sequelae.

The present inventor started as far as 1974 a different experience, taking into account the last portion of the prior paragraph, relating to the function. He thought that, when a function is not reached, the treatment was a failure, since in normal people function is always present. In order to give that normal function, a nail located in place must have the same or greater strength as the bone. On this premise every patient with tibia or femur fractures not only is able to have complete function, but also, due to the fact that said nail can support the body weight, including in the presence of lost bone, he or she can walk with total support (without crutches) three days after the surgical procedure. Bio-mechanic studies made at Montpellier (France) in Unit 103 of INSERM absolutely supported the above, and furthermore, this fact has been corroborated with more than 1600 patients intervened directly by the inventor. In order to obtain this, said nail must be solid, preferably of stainless steel 316 LS and with a diameter suitable for the weight of the patient. For instance, a 70 kg patient requires a 11 mm diameter nail for the femur. Or, for a patient with a weight of more than 80 kg, a 13 mm diameter bar is preferred. Up to the present time, the use of this embodiments of nails have had excellent results. Additionally, in place of screws, the inventor uses bolts, the strength of which is greater.

A new trend, started some years ago, is the use by certain commercial enterprises of the so-called latch-nail or blocked by means of screws. This nail is solid, of a very thin diameter so that it can be introduced into the pith channel without a need of pith milling, as commonly made for the previous ones. This type of nail, however solid, due to the fact that is so thin, has no the necessary strength to support the walking. So, the walking time for a patient must be delayed, as in the other instances, in order to prevent the rupture of the implant.

With all this in mind, the inventor designed a new nail, without milling, and also the instrumentation needed for the implantation thereof. This nail has the property of a strength greater than that of the present nails without millings.

### DISCLOSURE OF THE INVENTION

An intramedular nail is disclosed attached to the bone by means of proximal and distal bolts and side screws between nail and bone, without milling or with pith milling, and also the special instruments for implanting the same in fractures and fracture sequelae in femur, tibia and humerus.

The technical field to which this invention pertains is orthopedics and orthopedic traumatology.

The characteristic details of this novel nail and the special instrumentation for implanting the same, are clearly shown in the following disclosure and the drawings accompanying hereto as an exemplary illustration thereof; wherein the same reference characters refer to the same parts. In said drawings:
FIGURE 1 is a front perspective and profile view of the bolt and conical screw images.
FIGURE 2 is a side perspective view of the entire instrumental and a nail in place, together with a "piece to find out the nail orifices and notch" (5), an "instrument for stabilizing the nail and the piece to find out the nail orifices and notch" (4.0) and an "instrument showing exactly where the nail is located laterally" (10).
FIGURE 3 shows a "piece to find out the nail orifices and notch" (5) in a top view. It is also shown an "instrument for stabilizing the nail and the piece to find out the nail orifices and notch" in various different positions.
FIGURA 4 is a top view of the "piece to find out the nail orifices and notch" shown with its top incomplete, the middle complete and the bottom incomplete: In this figure the orifices are identified with literal: "A" corresponds to nail No. 1; "B" corresponds to nail No. 2, and so forth.
FIGURE 5 shows the "piece to find out the nail orifices and notch" in two positions: In the upper part in profile (5.1-1) and in the lower part as seen in a top view (5.1-2). Alto a "proximal drilling tower" is represented in 6 in this figure.
FIGURE 6 shows the bits to find out the nail orifices (9, 9.1), the bolt guide (7), the bit guide (8, 8.1), a piercing ram for soft tissues and reach the bone (17) and a measuring device to position the nail and the "piece to find out the nail orifices and notch" (18) parallel to each other.
FIGURE 7 illustrates a bit 9.3 and a scale 14. It is also shown an instrument to house said bit and scale No. 21. In 20 a measuring instrument is shown to be placed in the site of the bit; said instrument is concave at the distal portion thereof so as to follow the shape of the nail.
FIGURE 8 shows a front view of the "piece to find out the nail orifices and notch" (5.2), the "instrument showing exactly where the nail is located laterally" (10), and under No. 20 an "instrument to measure the distance from the nail to a firm place of the instrument to house the bit and the scale", No. 21; the point of this instrument is concave at the bottom thereof, so as to rest on the convex surface of the nail.

### DESCRIPTION OF THE FIGURES

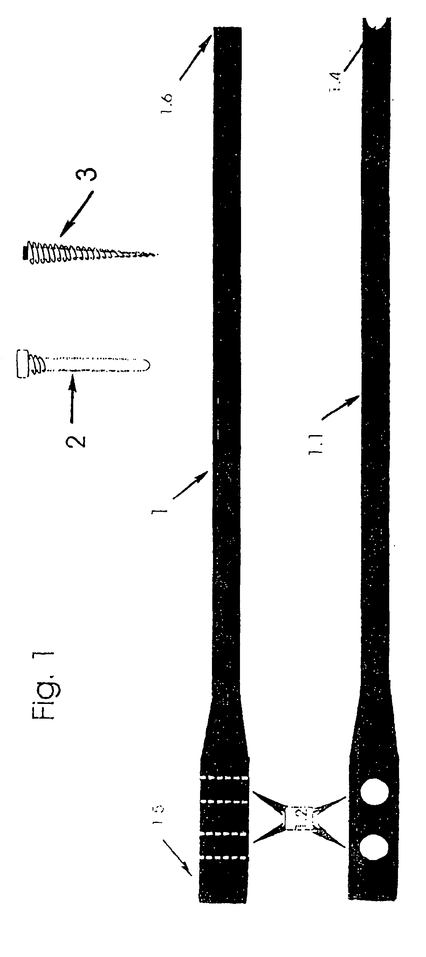

FIGURE 1.- It can be seen here the shape of the nails. A nail, generally identified by number 1, is seen at the anterior position; and by number 1.1 in a side position. Said nails have a zone that can be termed proximal (1.5), corresponding to the proximal portion of the patient. Said portion is broad and with the diameter thereof diminishing beyond the second orifice, where remains of the normal diameter for the remainder of the nail, down to its distal portion (1.6).

Said nails can be of various diameters and lengths, according to the needs of the patients, taking into account both the weight of the body and the diameter of the pith channel. Femur nails are straight; tibia and humerus nails have a proximal angle adapted to the inlet into said bones. In nail 1 there are proximal orifices (1.2), identified by shadowed spots. Under number 1.1, due to the side position thereof, said two proximal orifices (1.2) can be seen better, as well as the semi-tubular distal notch (1.4) serving as a housing for a distal bolt, as a solid support for the nail structure.

In can be seen also in said Figure 1 a bolt 2. As used in this disclosure and claims, a bolt is a screw with threads only on the proximal portion thereof, under the head, and the body of which is smooth, and the distal portion of which is rounded in order to prevent damages to the patient tissues. Said bolts are of different lengths and diameters; with a lower diameter at the proximal portion of the nail and a greater diameter for that corresponding to the nail notch or distal zone.

Also in Figure 1 and under No. 3, there is shown a completely conical screw, also of various lengths and diameters. The purpose thereof is to enter into a side of the nail, exerting pressure thereon and at the same time on the bone cortical, on both the distal fragment of the injury and the proximal one; whereby the nail stability within the pith channel increases remarkably.

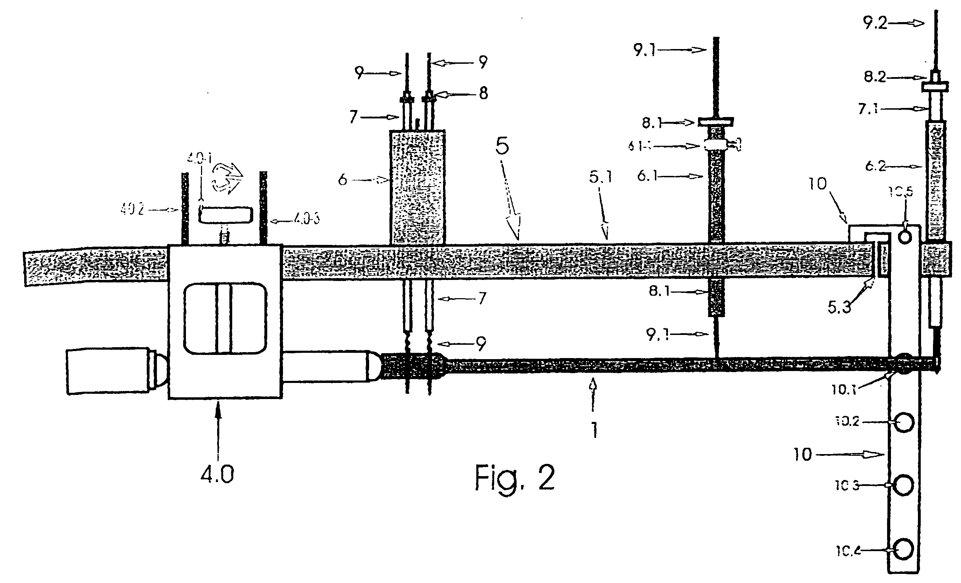

FIGURE 2 shows a side perspective of the instrumental and a nail positioned therein. Reference No. 1 is said nail. Reference No. 4.0 is the "instrument stabilizing the nail and the piece to find out the nail orifices and notch". Reference No. 5 is the "piece to find out the nail orifices and notch" and reference No. 10 is "the instrument showing exactly where the nail is laterally positioned" regarding the bone.

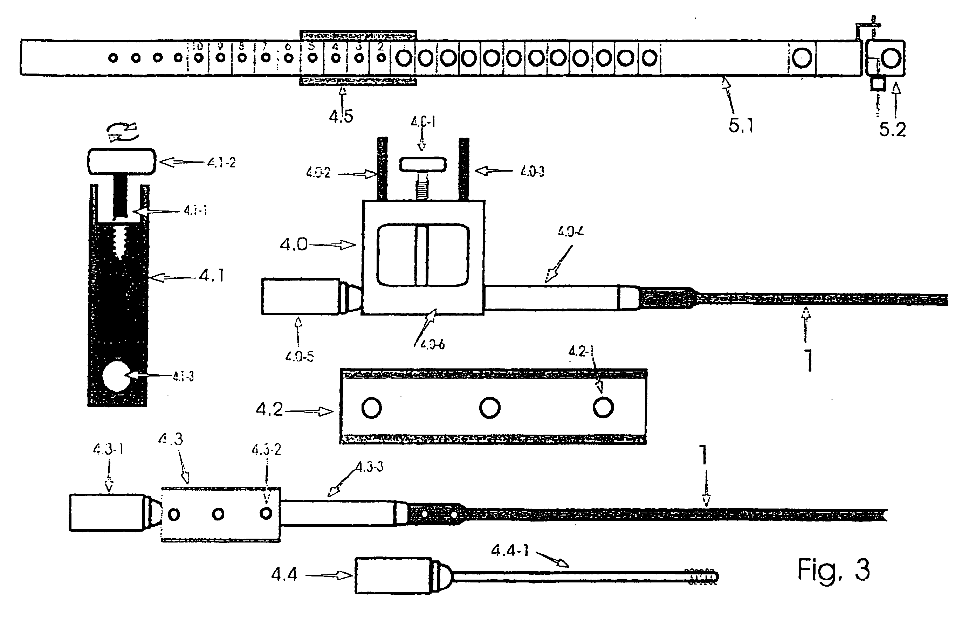

Said "instrument stabilizing the nail and the piece to find out the nail orifices and notch" will be broadly disclosed in Figure 3.

Said "piece to find out the nail orifices and notch" No. 5, is connected to the "instrument stabilizing the nail and the piece to find out the nail orifices and notch" by means of a screw 4.0-1 and in order to obtain a firmer and more secure connection, two pins, 4.0-2 and 4.03. As can be seen in this side view, said "piece" has various characteristics; for instance, it is divided into two portions: a proximal one identified by number 5.1 and the distal one, 5.2. Both portions are separated by a small space 5.3.

At the proximal portion of the "piece" is movably connected the "drilling tower for proximal orifices" (6). Said tower is placed on the precise site at the piece, according to the nail being used in the surgery, as will be clearly discussed hereinafter. Said tower is made with two tunnels wherein the "bolt guides" (7) enter and, in turn, the "bit guides" (8) enter these later and, within said guides the bits (9) enter. There can be also a "drilling tower for the distal notch" (6.2) with a sole tunnel wherein the "bolt guide" (7.1), "bit guide" (8.2) and bit (9.2) enter, and are of greater diameter than that of the "proximal tower". This "distal drilling tower" is firm.

A "tower to attain an exact parallelism between the piece to find out the nail orifices and notch" (6.1) is located between said two drilling tower. Through this tower a "bit guide" (8.1) and a thick bit (8.1) pass traversing the bone and reaching the nail just until it touches the surface thereof.

At the distal portion, near the "drilling tower for the distal notch" (6.2) a "system showing exactly where the nail is located laterally" (10) when the same is within the bone. This system is attached to the proximal portion (5.1) of the "piece to find out the nail orifices and notch". Said "system" can consist of four orifices (10.1, 10.2, 10.3 and 10.4) or more or less than four, at the will, but they must be related to the number of the "instruments stabilizing the nail and the piece to find out the nail orifices and notch". That is, if there are four "stabilizing instruments" of different heights, four orifices must be present in the "system showing exactly where the nail is located laterally". Further to said four orifices, another one is provided, slightly above the "instrument stabilizing the nail and the piece to find out the nail orifices and notch" (10.5). During the surgery, the orifice corresponding to the "stabilizer" employed will be used. Hereinbelow the upper or top orifice will be discussed.

FIGURE 3.- There are three elements, mainly the "instrument stabilizing the nail and the piece to find out the nail orifices and notch"; a nail and a "piece to find out the nail orifices and notch".

Said "Instrument stabilizing the nail and the piece to find out the nail orifices and notch" is consisted of a "handle" No. 4.0-5, 4.3-1 and 4.4, to be grasped by the surgeon. At the distal portion thereof there is a bar ending with a male thread, to attach the nails (4.4-1). Said "instrument" has also a "body" (4.0-6, 4.1, 4.2, 4.3, 4.5) the upper portion of which is a cavity in the shape of a rectangular pyramid No. 4.1-1 y 4,5, where said "piece to find out the nail orifices and notch" is introduce from above, within said cavity (4.5). The floor of said cavity is provided with three orifices of the same diameter (4.2-1 and 4.3-2) where, in order to attach and stabilize the same, a screw (4.0-1) and two bolts or pins (4.0-2 and 4.03) will enter. At the distal portion, No. 4.1, an orifice (4.1-3) appears corresponding to the instrument tunnel, through which the rod of the handle (4.4-1) passes. Finally, said "instrument" has a tube 4.0-4 and 4.3-3 housing a bar and wherein the threaded nail is introduced through said bar (4.4-1).

Various embodiments of the "instrument stabilizing the nail and the piece to find out the nail orifices and notch" No. 4.0 can be made. A small one will become near the patient skin during the surgery and others away thereof, useful regarding the thickness of the thigh, when a femur is involved, and the use of which makes easy the labor of the surgeon. Any of these instruments will be desirably positioned as near as possible to the skin.

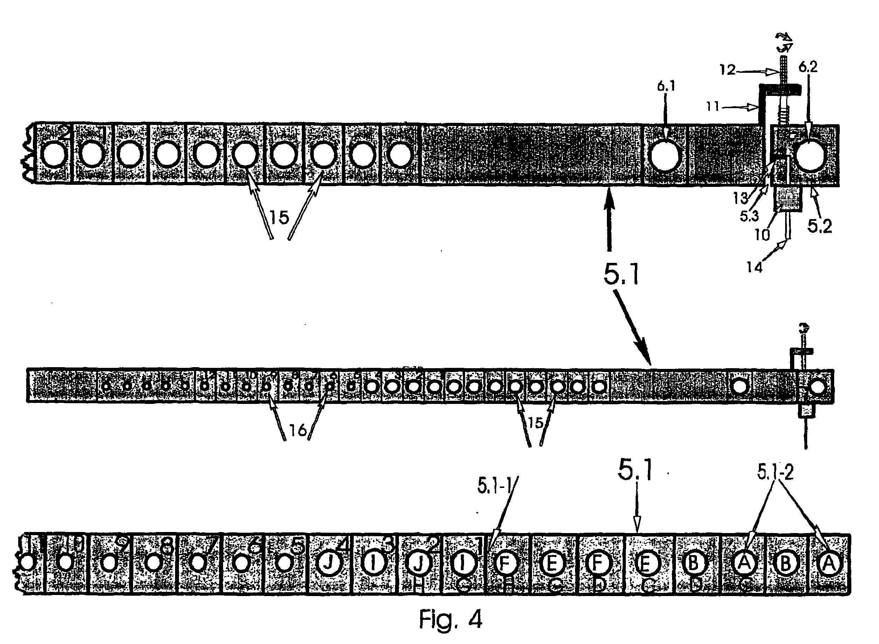

FIGURE 4. This is a top view of the "piece to find out the nail orifices and notch", which is incomplete at the top, complete at the middle and incomplete at the bottom. Letters have been assigned to the orifices thereof: "A" corresponding to nail No. 1; "B" to number 2, and so forth.

The distal portion 5.2 of the "piece to find out the nail orifices and notch" is moved by means of a screw No. 12, situated at an extension No. 11 of the proximal portion (5.1) of the "piece to find out the nail orifices and notch". Both said piece 11 and said screw 12 stabilize said distal portion 5.2 of said "piece to find out the nail orifices and notch".

The big orifices identified by number 15 and at the lower scheme identified by letters, are those where said "drilling tower for the proximal orifices" will be housed. Said tower is movable, according to the nail to be used. Said numbered orifices are identified with No. 16. Should a No. 1 nail is used, the strike in front of said number (5.1-1) must be placed at the distal portion of the "instrument stabilizing the nail and the piece to find out the nail orifices and notch", as can be appreciated in Figure 3 under No. 4.5. However, should a No. 2 nail is employed, the strike in front of the number will appear at the above mentioned place, and so forth, as also can be seen clearly in the side view of Figure 2.

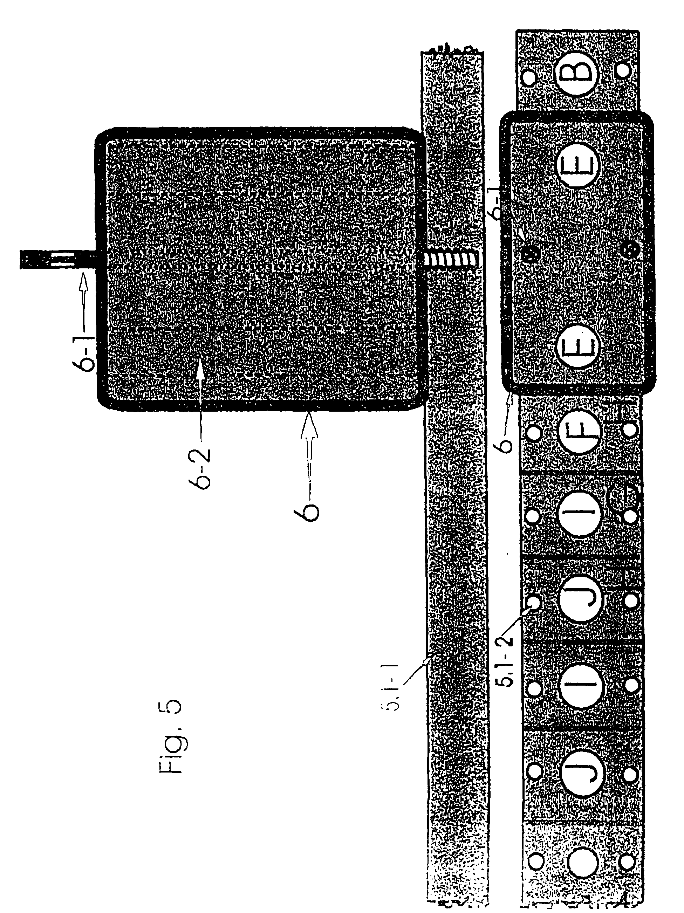

FIGURE 5. This figure shows said "piece to find out the nail orifices and notch" in two positions: in profile at the upper part (5.1-1) and at the lower part, as would appear in a top view (5.1-2). It is also represented a "proximal drilling tower" in number 6, the drilling channels of said "tower" under No. 6.2 and one of the two screws to attach the tower to the zone of those belonging to the "piece" where the big orifices are located, which are provided with two orifices having female threads.

Under No. 6.2 in Figure 5, two conducts are shown of said "tower" to locate the proximal orifices of said nail, and under No. 6-1 can be seen a long screw attaching said "tower" to the "piece to find out the nail orifices and notch". Said screw protrudes from the top portion, so as to allow for the surgeon to affix the same to the "guide", according to the number of the nail being employed. In 5.1-2 a top view of the "tower" is seen with the two conducts thereof bearing a letter E and said two screws 6-1-1, to attach said "tower" to every side of the "guide".

At the lower scheme said "tower" is so located that used the orifices "E" corresponding exactly to said nail No. 5, and is attached to said screws 6.1. Said "tower" is movable and takes the space of three orifices; although every number only has two orifices, there is always one of the orifices interposed of the following number.

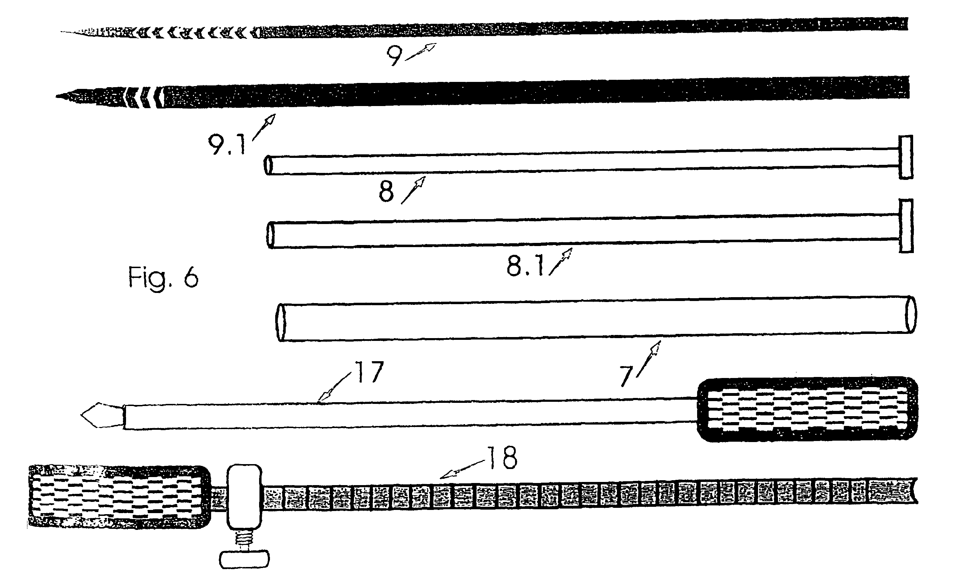

FIGURE 6.- This Figure shows the bits to locate the orifices of the nail 9 and 9.1 for parallelism and lower notch, the bolt guide (7), the bit guide (8, 8.1), the piercing tool to perforate soft tissues to reach the bone (17) and the measuring instrument to make said nail and said "piece to find out the nail orifices and notch" (18) parallel.

The above recited elements are shown in an exploded manner in Figure 6. Bit (9) belongs to the "proximal tower"; double parallelism bits, also belonging to the "distal tower" are represented by 9.1. The bit guides are: number 8, corresponding to the "proximal tower"; 8.1 corresponding to the "parallelism tower corresponding to the notch of said "distal tower "". The bolt guides (7) are for said "proximal tower" and bolts corresponding to distal tower are shown in Figure 2, numeral 7.1. Said "parallelism tower has not bolt guide. A piercing tool (17), entering through the bolt guide, and serving to traversing the soft tissues and reach the bone, once said guide reaches said bone, said piercing tool is withdrawn, the bit guide is arranged in place (8 or 8.1); the suitable bit (9 or 9.1) is introduced therein and the bone is drilled, passing through the nail orifices and the following bone cortical.

The above recited is also made with the "parallelism tower", but directly with the "tower". It is to be recall that, as already discussed, here there are no bolt guide, and only a cortical is drilled to reach the nail.

An instrument 18 is represented with a handle and a body numbered in millimeters or otherwise, entering said "parallelism tower". It has also a screw to be tightened in place, to an exact distance from the nail up to the bit guide exit, when this measure is taken before said nail is introduced into the patient member. A screw 6.1-1 in Figure 2 tightens the bit within said bit guide, in order to avoid any movement.

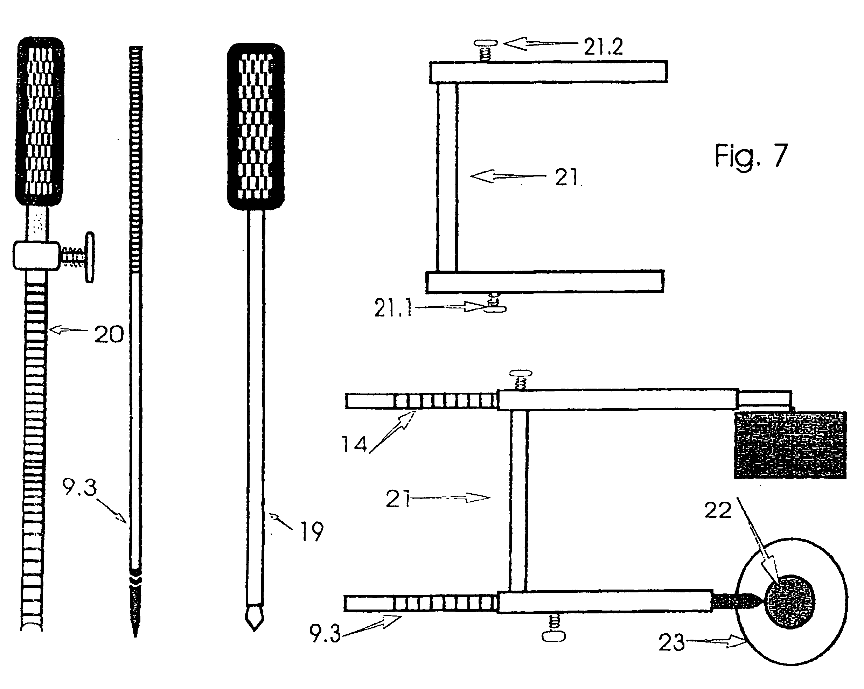

FIGURE 7.- As can be seen, a bit, 9.3 and a scale 14, are shown. There is a "parallel instrument" housing said bit and said scale 21. Figure 20 identifies an "instrument to measure the distance from the nail to a fixed site in the instrument that houses the bit end the scale", as shown in No. 18. Said instrument is arranged instead the measuring bit and is concave at the distal portion thereof so as to conform itself to the convex surface of the nail.

A piercing tool 19 is provided to be used during the surgery when the exact position of the nail within the bone must be determined, by means of the "nail locating instrument". Through the lower portion of the "instrument to connect the bit (9.3) and the scale (14)", signaling the site where the nail orifices are situated, a piercing tool is introduced down to the bone and is withdrawn. Then, a bore is drilled with a bit 9.3 and said instrument 20 is introduced, fastening the same by means of the screw provided at said "parallel instrument".

Should a number of different nail diameters are used, there will be a corresponding number or scales, since said piece has the measure given to said instrument No. 20, plus the distance from the surface to the nail center, that is also the center of the nail notch.

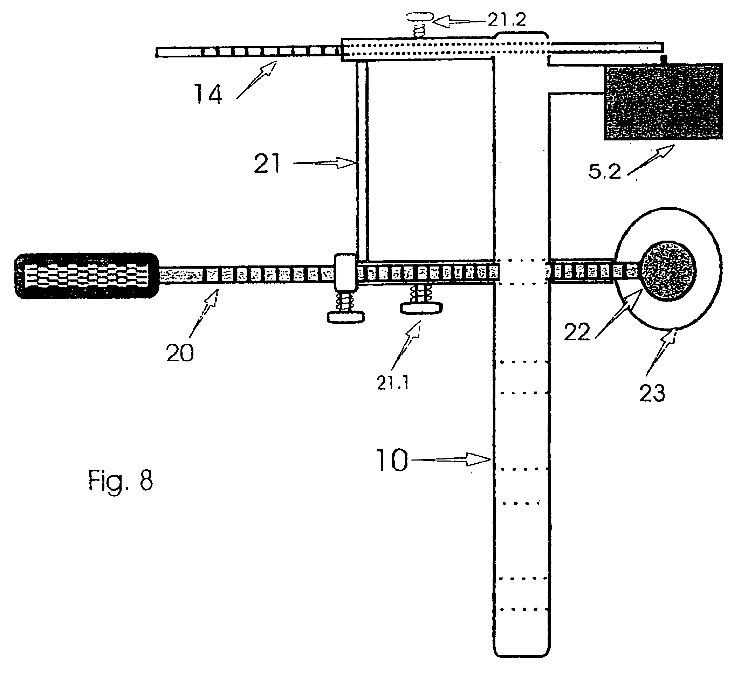

FIGURE 8.- This Figure is a front view of the "piece to find out the nail orifices and notch" (5.2), the "instrument showing exactly where the nail is located laterally", identified by numeral 10, and by numeral 20 the "instrument to measure the distance from the nail to a fixed site in the "instrument to housing a bit and a scale", No. 21; being the point of said instrument 20 concave at the lower portion, so as to bear on the convex surface of the nail.

### BEST METHOD TO CARRY OUT THE SURGERY WITH THIS INVENTION

Surgery can be carried out on tibia, femur or humerus, in fractures, lack of bonding of fractures, worse consolidated fractures and dismetries (a bone is shorter than other, when they must be equal) of the diaphysis of said bones,

Said nail with said bolts and conical screws can be used with fractures without a need of opening the focus thereof, or else, by opening said focus, at the surgeon decision.

Said nail enters the bones without a need for pith drilling, that is, without a need of broaden the pith channel. A greater diameter nail can be also used, which is highly resistant, with pith drilling, according to the needs of the patient and at the surgeon's decision. The nails without pith drilling are of higher strength comparing to those actually in the market place in the world over, and the nails with pith drilling are of lower diameter than any other in the actual market; being of great strength due to the characteristics thereof of having a distal notch in place of distal orifices.

### SURGICAL TECHNIQUES

At the surgical room, prior to the introduction of the nail into a patient, a nail will be placed on the instrumentalist table, to be used at the "instrument stabilizing the nail and the piece to find out the nail orifices and notch". Then, on the "piece to find out the nail orifices and notch" a "proximal drilling tower" is positioned at a suitable place, according to the number of nail to be used, and is fastened with the screws 6-1 of Figure 5, to the orifices of said "guide" 5.1-2 in the same Figure, when a No. 1 nail is employed, the "drilling tower" is situated on letter "A". Once this done, said "piece for orifices and notch" is arranged in the "stabilizing instrument", Figure 2 (4.0) and Figure 3 (4.0) at the cavity of the attaching instrument, Figure 3 (4.2-1) and 4.3-2, so as to become housed as seen in numeral 4.5. The line in front of the number of nail selected 5.1-1 in Figure 4, is positioned level to the distal portion of said cavity, as seen in numeral 4.5, Figure 3. Said "guide" is fastened by means of the screw 4.0-1 and said bolts 4.0-2 and 4.0-3, as seen in Figure 3.

Then it is basic that the correct measure when the nail and that the "guide of orifices and notch" are become perfectly parallel to each other, in order that, once the nail is within the bone, said parallelism can be exactly obtained. To this end, the parallelism instrument used in the nurse table, Figure 6, numeral 18, is taken and is introduced through the bit guide 8.1 of the "parallelism tower" 6.1 in Figure 2, until contacts said nail. Now the "instrument showing precisely where the nail is located laterally" is placed in position. Through the orifice of this instrument, corresponding to the "instrument to stabilize the nail and the piece to find out the nail orifices and notch", the instrument 20 of Figure 7 is introduced until the nail is reached. Should this latter cannot be attained, for instance, because the nail passes above or under said nail, said instrument 18 of Figure 7 is withdrawn a little until said instrument 20 is centered on the nail. The screw corresponding to every instrument is tightened and remains to the exact dimension from the "parallel instrument" 21 in Figures 7 and 8, to the outer surface of the nail. Said instrument 20 of Figure 7 is withdrawn now, with the screw tightened so as to keep the measure. The screw of instrument 18 of Figure 6 is then fastened in order to keep the exact measure for the parallelism.

Surgery of the patient's bone starts now.

The bone being treated is drilled proximately with a Küntscher piercer until the pith channel is reached. If no milling is made, the length of the nail desired to be used is made. When milling is made, the length of the nail is measured upon finishing the same.

With or without milling, the nail must enter the pith channel by turning the "stabilizing instrument" instead of by hammer blows; i. e., said nail must enter with certain freedom instead of forced. If this latter happens there can be three drawbacks: first, the nail cannot enter enough and becomes stuck therein; second, that upon hammering the nail the bone is broken due to the stuck position; and third, that when the nail is to be extracted, upon consolidation of the injury, said extraction becomes very hard to do. In general said nail can be introduced into the pith channel without the withdrawal of the "piece to find out the nail orifices and notch".

Once the nail inside, the proximal orifices thereof are localized by using the "proximal drilling tower", Figure 2, numeral 6. The "bolt guide" (7) is placed thereat and through this latter the "piercer" of Figure 6, numeral 17 is passed, so that the soft tissues are penetrated thereby together with the "bolt guide" until the cortical of the bone is reached. The piercer is withdrawn then and through the "bolt guide" the "bit guide" 8, Figure 2, is introduced and through the same a bit 9 that is rotated by a driller until the first cortical of the bone, the nail orifice and the second cortical of the bone are passed. The bit is leaved in order to stabilize with the nail itself the "piece to find out the nail orifices and notch". The same procedure is followed with the next proximal orifice and the bit is also leaved therein.

Next a test is made in order to determine whether the "piece for orifices and notch" and said nail are parallel and in the negative, the position is corrected. To this end a piercer 17, Figure 6, is passed through the "bit guide" 8.1, Figure 2, piercing the skin and the soft tissues, until the bone is reached. The piercer is then withdrawn and the bit 9.1, Figure 2 is positioned in the driller and is introduced through the bit guide 8.1, and the bone is drilled until the nail is reached. The bit is then withdrawn and the instrument 18, Figure 6, is carried into position until the screw thereof touches the bit guide and the nail, thus leaving this latter in parallel to the "piece to find out orifices and notch" and the instrument is fastened by means of the screw 6.1-1, Figure 2.

The distal notch of the nail can be then located. It can happen that the nail, upon entering the bone drifts away and then the notch of the nail and the bit 9.2 of the "distal drilling tower" 6.2, Figure 2, do not register. This is why the surgeon needs the "instrument showing exactly where the nail is located laterally", Figure 2, numeral 10; and also the parallelism between the nail and the "piece to find out the orifices and notch" in order he to know exactly where the nail is within the bone.

Thanks to the "instrument showing exactly where the nail is located laterally", numeral 10, Figure 8, and to the orifice of the latter corresponding to the "stabilizing instrument" the surgeon is able to situate the "instrument to house the bit and the scale" numeral 21, provided in said orifice of "instrument 10" and the upper portion thereof in the upper orifice of the same instrument. Next the piercer 19, Figure 7 is introduced, through the skin and soft tissues until the bone is reached; is withdrawn and is replaced by a bit 9.3, to drill the bone until the nail is reached, as shown in Figure 7. Said bit is then withdrawn and the instrument 20 is positioned as shown in Figure 8, wherein a measure is already kept by the screw thereof and, in turn, when the nail is touched, the same is fastened by means of the screw in the "instrument to house the bit and the scale" 21.

The scale corresponding to the diameter of the nail being used is passed then through the upper orifice of this instrument 21, in such a manner that the rear portion thereof has the same length as the instrument 20, but at the body thereof, never at the handle (recall that both are numbered). Said scale is fastened by a screw provided at the upper tube of the instrument 21. The surgeon finds then where the point of the scale is, because in that point is the nail notch. If this latter registers the mark made at the "piece to find out the nail orifices and notch", as can be seen in Figure 4, numeral 13, corresponding to the distal portion of said "piece" 5.2 in the same Figure, the drilling will be made by the "distal drilling tower"; but in case said scale and the mark 13 of Figure 4 do not register, the distal portion 5.2 of the "piece to find out the nail orifices and notch" is moved by means of the screw 12 of the same Figure, until both register exactly, and then the drilling is made.

In order to drill with the "distal tower", the bolt guide 7.1 is placed inside the tower; the piercer 17, Figure 6 is introduced through the soft tissues until the bone is reached. Then the piercer is withdrawn and the bit guide 8.2 of the same Figure is positioned, and then the bit 9.2 is introduced beyond the first cortical, the notch and the second cortical of the bone. Said bit is withdrawn and the length of the 5 mm diameter bolt (or any other diameter) is measured and then introduced by means of an Allen wrench, the bit guide is withdrawn and the bolt is introduced through the bolt guide beyond the first cortical, the notch until exits through the second cortical of the bone, and is screwed to said first cortical.

The most distal bit 9 of the "proximal drilling tower" is then withdrawn; the bit guide is withdrawn, the thinner bolt of 4 mm or any other diameter is measured and positioned in the same manner as the previous one. The same steps are taken in the more proximal orifice of the nail and the osteosynthesis is finished. Then radiographies are made, the instrumental is withdrawn, a canalization is left in place and the soft tissues are closed.

Having thus described the invention, what the inventor considers as a novelty and therefore, claims as his exclusive property is contained in the following claims.

## Claims

1. A new high strength intramedular nail bolt fastened to the bone, with or without milling, **characterized in that** a distal notch is provided instead of the orifices provided in the nails presently available in the market; said notch is a distal support point of the nail, of high strength due to the fact that carries a thick bolt; contrary to the nails without milling, that are thinner, and their strength is minimal due to the presence of distal orifices, the nail of this invention is more resistant, due to the fact that there are no orifices through which the nail can be broken, but only a notch capable of stabilizing the injury but not prone to breakage.

2. A new high strength intramedular nail bolt fastened to the bone, with or without milling, **characterized in that** conical screws are provided of different lengths and diameters, capable of being adapted to every patient, never mentioned in the present latched fastening systems; said screws increase the stability between bone and nail, due to the fact that they are pressure introduced thereinto and extend in the space existing therebetween, and become firmly tightened against the nail and the cortical; this fact is more clearly visible when a screw enters through one side in the upper fragment of the bone and other one through the contrary side in the lower fragment thereof.

3. A new high strength intramedular nail bolt fastened to the bone, with or without milling, **characterized in that** an instrumental is used comprising a piece to find out the nail orifices and notch, together with three types of drilling towers: proximal, distal and for parallelism; with said proximal drilling tower being capable of movement to give the desired number of nail and attaches to said piece, instead of being the distal tower the one that is displaced, whereby important problems are avoided.

4. A new high strength intramedular nail bolt fastened to the bone, with or without milling, **characterized in that** said distal tower bit is of greater diameter in order to allow for the entrance in the notch of a bolt thicker than the ones for the proximal orifices, whereby the entire system becomes of greater strength.

5. A new high strength intramedular nail bolt fastened to the bone, with or without milling, **characterized in that** the system for the parallelism between the nail and the piece to find out the nail orifices and notch comprises a parallelism tower, a bit guide, a bit, a fastening screw of the measuring instrument to leave the nail and the piece to find out the nail orifices and notch parallel to each other; none of which is disclosed in the literature, with said elements providing the easy and correct function of the instrument showing exactly where the nail is located laterally: as if said pieces were not parallel to each other, the bit could pass above or below the nail when drilling in search thereof; there being pieces for the security of the system, comprising a numbered bit; a scale entering at the top portion of the instrument; and an instrument to be positioned when the measuring bit is withdrawn, with the correct given measurement as taken prior to the starting of the surgery in a patient.

6. A new high strength intramedular nail bolt fastened to the bone, with or without milling, **characterized in that** at least four models of the instrument for stabilization of the nail and the piece to find out the nail orifices and notch; which instruments can be short for lean people and become greater until they can be used with obese people; this fact allows that the surgeon can operate near the skin of the patient, thus giving more security to the system.

7. A new high strength intramedular nail bolt fastened to the bone, with or without milling, **characterized in that** said piece to find out the nail orifices and notch enters from above in the instrument stabilizing the nail and the piece to find out the nail orifices and notch; whereby the surgery is made highly easier when placing or removing a piece, mainly when a tibia or a humerus is involved, since, in general, this type of pieces are slided through the instrument.

8. A new high strength intramedular nail bolt fastened to the bone, with or without milling, **characterized in that** said piece to find out the nail orifices and notch is fastened by means of a screw and two bolts or pins to the instrument stabilizing the nail and the piece to find out the nail orifices and notch.

9. Finally, the complete instrument is claimed which is used to place this nail of femur, tibia and humerus.
